# EUROPEAN PATENT APPLICATION

(11) **EP 4 163 395 A1**
(43) Date of publication of application: **12.04.2023**
(21) Application number: 21818700.3
(22) Date of filing: 05.04.2021
(51) Int. Cl.: C12Q 1/6888

(54) **SYSTEM AND METHOD FOR IDENTIFYING SPECIES OR SUBSPECIES OF NON-TUBERCULOUS MYCOBACTERIA PRESENT IN SPECIMEN**

(30) Priority: 03.06.2020 JP 2020096551
(71) Applicant: Bikenbiomics Co., Ltd., Ibaraki-shi, Osaka 567-0041 (JP)
(72) Inventor: NAKAMURA Noboru, Ibaraki-shi, Osaka 567-0041 (JP); NAKAMURA Shota, Suita-shi, Osaka 565-0871 (JP); MATSUMOTO Yuki, Suita-shi, Osaka 565-0871 (JP); KINJO Takeshi, Nakagami-gun, Okinawa 903-0125 (JP)
(74) Representative: Greaves Brewster LLP
(86) International application number: PCT/JP2021/014475
(87) International publication number: WO 2021/246045

(57) **Abstract**

The present disclosure makes it possible to accurately and rapidly identify, with respect to a specimen including non-tuberculous mycobacteria (NTM), species and subspecies of NTM contained in the specimen. A system according to the present disclosure includes a sequencing device for acquiring sequence information of multiple genes in the genomic sequence of NTM present in the specimen; a database for storing sequence information of multiple genes in known NTM; and a software for comparing the sequence information of the multiple genes acquired by the sequencing device with the sequence information of the multiple genes stored in the database to select, from the known NTM stored in the database, species or subspecies of NTM having most similar sequence information to the sequence information of NTM in the specimen.

## Description

### TECHNICAL FIELD

The present disclosure relates to a system and a method for identifying species or subspecies of non-tuberculous mycobacteria in a specimen.

### BACKGROUND ART

Bacteria of the genus Mycobacterium are commonly known to cause various infections and are in general classified in three groups, which are Mycobacterium tuberculosis causing tuberculosis; Mycobacterium leprae responsible for Hansen's disease; and non-tuberculous mycobacteria (NTM) other than Mycobacterium tuberculosis and Mycobacterium leprae.

While having been established for tuberculosis and Hansen's disease, preventive and therapeutic methods have not yet sufficiently established for respiratory infections (NTM disease) mainly caused by NTM. For that reason, NTM disease is recognized as an infection of importance in public health. In recent years, increased morbidity in NTM disease has been also reported (see, for example, Non-patent document 1).

Identification of species and subspecies of pathogenic bacteria is of first importance for determining therapeutic strategies for NTM disease, and acid-fast staining and mass spectrometry were conventionally used for the identification. In recent years, MLST (multi-locus sequence typing) is particularly used for the identification, and MLST includes performing DNA sequencing of multiple housekeeping genes of a specimen and comparing the resulting sequences with DNA sequences registered in a database (see, for example, Non-patent documents 2, 3). In some methods based on genomic information, species of bacteria are successfully identified at the subspecies level by using ribosomal genes and core and whole genome information (see, for example, Non-patent documents 4 to 6).

### CITATION LIST

### NON-PATENT DOCUMENT

[Non-patent Document 1] Namkoong H. et al. Emerg Infect Dis. 2016, 22, 1116-1117
[Non-patent Document 2] Stackebrandt E. et al. Int J Syst Evol Microbiol. 1994, 44, 846-849
[Non-patent Document 3] Enright MC. et al. Trends Microbiol. 1999, 7, 482-487
[Non-patent Document 4] Maiden MCJ. et al. Nat Rev Microbiol. 2013, 11, 728-736
[Non-patent Document 5] Zolfo M. et al. Nucleic Acid Res. 2016, 1, 1-10
[Non-patent Document 6] Jolley KA. et al. BMC Bioinformatics. 2010, 11, 595

### SUMMARY OF INVENTION

### TECHNICAL PROBLEM

When MLST is used, it is necessary as described above to compare the DNA sequence of the specimen and the DNA sequences of bacterial species registered in the database. In this respect, the existing databases still do not have sufficient registered genomic information and identifiable bacterial species are limited. Processes for MLST, such as sequencing of specimens and sequence comparison, also need a significant amount of time even when expensive sequencing and computing devices are used. It thus requires a large amount of time to identify species and subspecies of pathogenic bacteria for multiple specimens in order to diagnose NTM disease, resulting in difficulty in practical applications.

The present disclosure is made in view of the foregoing problems, and an object of the present disclosure is to accurately and rapidly identify, with respect to a specimen including NTM, species and subspecies of NTM contained in the specimen.

### SOLUTION TO PROBLEM

The inventors have found, as the result of an earnest study, genes that can be effectively used as indicators for accurately and rapidly identifying species and subspecies of NTM using MLST, and developed the present disclosure.

Specifically, a system according to the present disclosure is a system for identifying species or subspecies of NTM in a specimen using MLST (multi-locus sequence typing), comprising a sequencing device for acquiring sequence information of multiple genes in the genomic sequence of NTM present in the specimen; a database for storing sequence information of known NTM; and a computing device for comparing the sequence information of the multiple genes acquired by the sequencing device with sequence information of the multiple genes stored in the database to select, from the known NTM stored in the database, species or subspecies of NTM having most similar sequence information to the sequence information of NTM in the specimen, wherein the multiple genes are selected from the group consisting of 184 genes shown in Table 1.

According to the system of the present disclosure, desired species or subspecies of NTM are identified using MLST by utilizing the sequence information of said multiple genes that are necessary and sufficient to identify species or subspecies of a large number of NTM using MLST, thus enabling very accurate and rapid identification of the desired species or subspecies of NTM.

In the system of the present disclosure, the multiple genes may preferably include 30S ribosome S1 to S21 (rpsA to rpsU), 50S ribosome L1 to L36 (rplAto rplF, rplI to rplU, rpmA to rpmJ), 5S ribosome RNA (rrf), 16S ribosome RNA (rrs), 23S ribosome RNA (rrl), rpoB, groEL2, gyrB, inhA, tlyA, katG, pncA, erm, eis, and embB.

In the system of the present disclosure, the database may be preferably mlstdb. NTM (https://github.com/ymatsumoto/mlstverse.Mycobacterium.db).

In the system of the present disclosure, the software may be preferably mlstverse (http s: //github. com/ymatsumoto/ml stverse).

A method according to the present disclosure is a method for identifying species or subspecies of NTM in a specimen using MLST (multi-locus sequence typing), comprising the steps of: performing sequencing of NTM present in the specimen to acquire sequence information of multiple genes in the genomic sequence of NTM; comparing the acquired sequence information with sequence information of the multiple genes in known NTM stored in a predetermined database; and selecting, from the known NTM stored in the database, species or subspecies of NTM having most similar sequence information to the sequence information of NTM in the specimen, by using a result of the comparison between the sequence information of the multiple genes, wherein the multiple genes are selected from the group consisting of 184 genes shown in Table 1.

According to the method of the present disclosure, desired species or subspecies of NTM are identified using MLST by utilizing the sequence information of said multiple genes serving as multiple genes necessary and sufficient to identify species or subspecies of a large number of NTM using MLST, thus enabling very accurate and rapid identification of the desired species and subspecies of NTM, as is the case with the system of the present disclosure.

The method of the present disclosure may preferably include 30S ribosome S1 to S21 (rpsAto rpsU), 50S ribosome L1 to L36 (rplAto rplF, rplI to rplU, rpmAto rpmJ), 5S ribosome RNA (rrf), 16S ribosome RNA (rrs), 23 S ribosome RNA (rrl), rpoB, groEL2, gyrB, inhA, tlyA, katG, pncA, erm, eis, and embB.

In the method of the present disclosure, the database may be preferably mlstdb. NTM (https://github.com/ymatsumoto/mlstverse.Mycobacterium.db).

In the method of the present disclosure, the software may be preferably mlstverse (http s: //github. com/ymatsumoto/ml stverse).

### ADVANTAGEOUS EFFECTS OF INVENTION

According to the system and method of the present disclosure for identifying species or subspecies of NTM in a specimen, species and subspecies of NTM in the specimen containing various NTM can be accurately and rapidly identified.

### BRIEF DESCRIPTION OF DRAWINGS

Figure 1 shows a workflow for a software used in a system for identifying species or subspecies of NTM in a specimen, according to an embodiment of the present disclosure.
Figure 2 shows photographs of 29 samples isolated from clinical specimens in Examples.

### DESCRIPTION OF EMBODIMENTS

Embodiments of the present disclosure are described below. The following description of preferred embodiments is merely an example in nature, and is not intended to limit the present disclosure, application methods of the present disclosure, or use of the present disclosure.

A system for identifying species or subspecies of NTM in a specimen, according to an embodiment of the present disclosure, uses MLST (multi-locus sequence typing) and includes a sequencing device for acquiring sequence information of multiple genes in the genomic sequence of NTM present in the specimen; a database for storing sequence information of multiple genes in known NTM; and a computing device for comparing the sequence information of the multiple genes acquired by the sequencing device with sequence information of the multiple genes stored in the database to select, from the known NTM stored in the database, species or subspecies of NTM having most similar sequence information to the sequence information of NTM in the specimen. The multiple genes are then selected from the group consisting of 184 genes shown in Table 1 explained below.

A method for identifying species or subspecies of NTM in a specimen, according to an embodiment of the present disclosure, is a method for identifying species or subspecies of non-tuberculous mycobacteria (NTM) in a specimen using MLST (multi-locus sequence typing), the method including the steps of: performing sequencing of NTM present in the specimen to acquire sequence information of multiple genes in the genomic sequence of NTM; comparing the acquired sequence information with sequence information of the multiple genes in known NTM stored in a predetermined database; and selecting, from the known NTM stored in the database, species or subspecies of NTM having most similar sequence information to the sequence information of NTM in the specimen, by using a result of the comparison of the sequence information of the multiple genes. The multiple genes are selected from the group consisting of 184 genes shown in Table 1 explained below.

MLST is a method used for classifying, for example, bacteria. MLST is a method for determining the base sequences of DNAs included in multiple genes in bacteria of interest, comparing the base sequences to a database storing base sequence information of multiple genes in known bacteria, and thereby classifying and identifying the bacteria of interest. Generally, multiple essential genes, such as housekeeping genes, are often used as the multiple genes. For example, when seven genes are used in MLST, the base sequences of the seven essential genes of interest having a 100 percent match to the base sequences of the seven essential genes in a sequence type (ST) of a certain bacterial species, allows considering as having the same sequence type to classify as this bacterial species. When even one of the base sequences of the seven genes differs, the bacteria is considered as a subspecies to be classified as a different species.

In the embodiment, the specimen includes, but not limited to, a biological sample including, for example, body fluid, such as blood, lymph fluid, obtained from a human and an animal. The specimen may be a solution containing NTM, which is obtained by purifying NTM from the biological sample and containing NTM in a predetermined vehicle. Particularly, the specimen may be preferably a solution containing only one type of NTM isolated by using a known bacteria isolation method, such as dilution plating method.

In the embodiment, genomic DNA in a specimen is extracted before acquiring sequence information of multiple genes in the genomic sequence of NTM present in the specimen. Methods for extracting genomic DNA are known to a person of ordinary skill in the art and an optimal method according to a specimen is appropriately selected by a person skilled in the art. The obtained genomic DNA is subject to pretreatment known to a person skilled in the art, such as fragmentation, to allow sequence determination by a sequencing device.

In the embodiment, whilst the sequencing device for acquiring sequence information of multiple genes in the genomic sequence of NTM in a specimen is not particularly limited as long as the device can determine base sequences, the device may be preferably a device able to more quickly and accurately determine base sequences. The sequencing device may be preferably, for example, a sequencing device able to simultaneously determine the base sequences of multiple specimens or to obtain results of determined base sequences in real time. Sequencing devices, such as Miseq available from Illumina and MinION available from Oxford Nanopore Technologies, may be preferably used.

In the embodiment, the database for storing sequence information of multiple genes of known NTM is not particularly limited as long as the database is a published MLST database including base sequence information of many species of NTM. Particularly, mlstdb.NTM (https://github.com/ymatsumoto/mlstverse.Mycobacterium.db) created by the inventors may be preferably used. mlstdb.NTM has been created using genomic information of 175 NTM species and stores sequence information of 184 genes related to, for example, ribosomal proteins, ribosomal RNA genes, antibiotic resistance genes, and virulence genes. Particularly, mlstdb.NTM stores sequence types for sequences of the 184 genes, corresponding to species or subspecies of NTM. The 184 genes are as shown in Table 1 below.

**Table 1**

| No. | Gene name | Product | Category |
|---|---|---|---|
| 1 | rpsA | 30S ribosomal protein S1 | RP |
| 2 | rpsB | 30S ribosomal protein S2 | RP |
| 3 | rpsC | 30S ribosomal protein S3 | RP |
| 4 | rpsD | 30S ribosomal protein S4 | RP |
| 5 | rpsE | 30S ribosomal protein S5 | RP |
| 6 | rpsF | 30S ribosomal protein S6 | RP |
| 7 | rpsG | 30S ribosomal protein S7 | RP |
| 8 | rpsH | 30S ribosomal protein S8 | RP |
| 9 | rpsI | 30S ribosomal protein S9 | RP |
| 10 | rpsj | 30S ribosomal protein S10 | RP |
| 11 | rpsK | 30S ribosomal protein S11 | RP |
| 12 | rpsL | 30S ribosomal protein S12 | RP |
| 13 | rpsM | 30S ribosomal protein S13 | RP |
| 14 | rpsN | 30S ribosomal protein S14 | RP |
| 15 | rps0 | 30S ribosomal protein S15 | RP |
| 16 | rpsP | 30S ribosomal protein S16 | RP |
| 17 | rpsQ | 30S ribosomal protein S17 | RP |
| 18 | rpsR | 30S ribosomal protein S18 | RP |
| 19 | rpsS | 30S ribosomal protein S19 | RP |
| 20 | rpst | 30S ribosomal protein S20 | RP |
| 21 | rpsU | 30S ribosomal protein S21 | RP |
| 22 | rplA | 50S ribosomal protein L1 | RP |
| 23 | rplB | 50S ribosomal protein L2 | RP |
| 24 | rplC | 50S ribosomal protein L3 | RP |
| 25 | rplD | 50S ribosomal protein L4 | RP |
| 26 | rplE | 50S ribosomal protein L5 | RP |
| 27 | rplF | 50S ribosomal protein L6 | RP |
| 28 | rplL | 50S ribosomal protein L7/L12 | RP |
| 29 | rplI | 50S ribosomal protein L9 | RP |
| 30 | rplJ | 50S ribosomal protein L10 | RP |
| 31 | rplK | 50S ribosomal protein L11 | RP |
| 32 | rplM | 50S ribosomal protein L13 | RP |
| 33 | rplN | 50S ribosomal protein L14 | RP |
| 34 | rp10 | 50S ribosomal protein L15 | RP |
| 35 | rplP | 50S ribosomal protein L16 | RP |
| 36 | rplQ | 50S ribosomal protein L17 | RP |
| 37 | rplR | 50S ribosomal protein L18 | RP |
| 38 | rplS | 50S ribosomal protein L19 | RP |
| 39 | rplT | 50S ribosomal protein L20 | RP |
| 40 | rplU | 50S ribosomal protein L21 | RP |
| 41 | rplV | 50S ribosomal protein L22 | RP |
| 42 | rplW | 50S ribosomal protein L23 | RP |
| 43 | rplX | 50S ribosomal protein L24 | RP |
| 44 | rpmA | 50S ribosomal protein L27 | RP |
| 45 | rpmB | 50S ribosomal protein L28 | RP |
| 46 | rpmC | 50S ribosomal protein L29 | RP |
| 47 | rpmD | 50S ribosomal protein L30 | RP |
| 48 | rpmE | 50S ribosomal protein L31 | RP |
| 49 | rpmF | 50S ribosomal protein L32 | RP |
| 50 | rpmG | 50S ribosomal protein L33 | RP |
| 51 | rpmH | 50S ribosomal protein L34 | RP |
| 52 | rpmI | 50S ribosomal protein L35 | RP |
| 53 | rpmj | 50S ribosomal protein L36 | RP |
| 54 | Rv0440 | hsp65 | hsp65 |
| 55 | Rv0667 | rpoB | AMR |
| 56 | rrs | 16S ribosomal RNA | AMR |
| 57 | rrl | 23S ribosomal RNA | AMR |
| 58 | Rv0005 | gyrB | AMR |
| 59 | Rv0006 | gyrA | AMR |
| 60 | Rv1484 | inhA | AMR |
| 61 | Rv1694 | tlyA | AMR |
| 62 | Rv1908c | katG | AMR |
| 63 | Rv1988 | erm | AMR |
| 64 | Rv2043c | pncA | AMR |
| 65 | Rv2416c | eis | AMR |
| 66 | Rv3795 | embB | AMR |
| 67 | Rv3614c | | ESX-1 (T7SS) |
| 68 | Rv3615c | | ESX-1 (T7SS) |
| 69 | Rv3616c | | ESX-1 (T7SS) |
| 70 | Rv3849 | | ESX-1 (T7SS) |
| 71 | Rv3862c | | ESX-1 (T7SS) |
| 72 | Rv3864 | | ESX-1 (T7SS) |
| 73 | Rv3865 | | ESX-1 (T7SS) |
| 74 | Rv3866 | | ESX-1 (T7SS) |
| 75 | Rv3867 | | ESX-1 (T7SS) |
| 76 | Rv3868 | | ESX-1 (T7SS) |
| 77 | Rv3869 | | ESX-1 (T7SS) |
| 78 | Rv3870 | | ESX-1 (T7SS) |
| 79 | Rv3871 | | ESX-1 (T7SS) |
| 80 | Rv3872 | | ESX-1 (T7SS) |
| 81 | Rv3873 | | ESX-1 (T7SS) |
| 82 | Rv3874 | | ESX-1 (T7SS) |
| 83 | Rv3875 | | ESX-1 (T7SS) |
| 84 | Rv3876 | | ESX-1 (T7SS) |
| 85 | Rv3877 | | ESX-1 (T7SS) |
| 86 | Rv3878 | | ESX-1 (T7SS) |
| 87 | Rv3879c | | ESX-1 (T7SS) |
| 88 | Rv3880c | | ESX-1 (T7SS) |
| 89 | Rv3881c | | ESX-1 (T7SS) |
| 90 | Rv3882c | | ESX-1 (T7SS) |
| 91 | Rv3883c | | ESX-1 (T7SS) |
| 92 | Rv3884c | | ESX-2 (T7SS) |
| 93 | Rv3885c | | ESX-2 (T7SS) |
| 94 | Rv3886c | | ESX-2 (T7SS) |
| 95 | Rv3887c | | ESX-2 (T7SS) |
| 96 | Rv3889c | | ESX-2 (T7SS) |
| 97 | Rv3890c | | ESX-2 (T7SS) |
| 98 | Rv3891c | | ESX-2 (T7SS) |
| 99 | Rv3892c | | ESX-2 (T7SS) |
| 100 | Rv3893c | | ESX-2 (T7SS) |
| 101 | Rv3894c | | ESX-2 (T7SS) |
| 102 | Rv3895c | | ESX-2 (T7SS) |
| 103 | Rv0282 | | ESX-3 (T7SS) |
| 104 | Rv0283 | | ESX-3 (T7SS) |
| 105 | Rv0284 | | ESX-3 (T7SS) |
| 106 | Rv0285 | | ESX-3 (T7SS) |
| 107 | Rv0286 | | ESX-3 (T7SS) |
| 108 | Rv0287 | | ESX-3 (T7SS) |
| 109 | Rv0288 | | ESX-3 (T7SS) |
| 110 | Rv0289 | | ESX-3 (T7SS) |
| 111 | Rv0290 | | ESX-3 (T7SS) |
| 112 | Rv0291 | | ESX-3 (T7SS) |
| 113 | Rv0292 | | ESX-3 (T7SS) |
| 114 | Rv3444c | | ESX-4 (T7SS) |
| 115 | Rv3445c | | ESX-4 (T7SS) |
| 116 | Rv3447c | | ESX-4 (T7SS) |
| 117 | Rv3448 | | ESX-4 (T7SS) |
| 118 | Rv3449 | | ESX-4 (T7SS) |
| 119 | Rv3450c | | ESX-4 (T7SS) |
| 120 | Rv1782 | | ESX-5 (T7SS) |
| 121 | Rv1783 | | ESX-5 (T7SS) |
| 122 | Rv1785c | | ESX-5 (T7SS) |
| 123 | Rv1787 | | ESX-5 (T7SS) |
| 124 | Rv1788 | | ESX-5 (T7SS) |
| 125 | Rv1789 | | ESX-5 (T7SS) |
| 126 | Rv1790 | | ESX-5 (T7SS) |
| 127 | Rv1791 | | ESX-5 (T7SS) |
| 128 | Rv1793 | | ESX-5 (T7SS) |
| 129 | Rv1795 | | ESX-5 (T7SS) |
| 130 | Rv1796 | | ESX-5 (T7SS) |
| 131 | Rv1797 | | ESX-5 (T7SS) |
| 132 | Rv1798 | | ESX-5 (T7SS) |
| 133 | Rv2430c | | ESX-5 (T7SS) |
| 134 | Rv2431c | | ESX-5 (T7SS) |
| 135 | Rv3097c | | ESX-5 (T7SS) |
| 136 | Rv0176 | | Mce associated transmembrane protein |
| 137 | Rv0177 | | Mce associated transmembrane protein |
| 138 | Rv0167 | | Mcel |
| 139 | Rv0168 | | Mcel |
| 140 | Rv0169 | | Mcel |
| 141 | Rv0170 | | Mcel |
| 142 | Rv0171 | | Mcel |
| 143 | Rv0172 | | Mcel |
| 144 | Rv0173 | | Mcel |
| 145 | Rv0174 | | Mcel |
| 146 | Rv0589 | | Mce2 |
| 147 | Rv0591 | | Mce2 |
| 148 | Rv0592 | | Mce2 |
| 149 | Rv0593 | | Mce2 |
| 150 | Rv0594 | | Mce2 |
| 151 | Rv1966 | | Mce3 |
| 152 | Rv1967 | | Mce3 |
| 153 | Rv1968 | | Mce3 |
| 154 | Rv1969 | | Mce3 |
| 155 | Rv1970 | | Mce3 |
| 156 | Rv1971 | | Mce3 |
| 157 | Rv3494c | | Mce4 |
| 158 | Rv3495c | | Mce4 |
| 159 | Rv3496c | | Mce4 |
| 160 | Rv3497c | | Mce4 |
| 161 | Rv3498c | | Mce4 |
| 162 | Rv3499c | | Mce4 |
| 163 | Rv0754 | | PE/PPE protein |
| 164 | Rv0834c | | PE/PPE protein |
| 165 | Rv0978c | | PE/PPE protein |
| 166 | Rv1169c | | PE/PPE protein |
| 167 | Rv1818c | | PE/PPE protein |
| 168 | Rv2396 | | PE/PPE protein |
| 169 | Rv3508 | | PE/PPE protein |
| 170 | Rv3511 | | PE/PPE protein |
| 171 | Rv3514 | | PE/PPE protein |
| 172 | Rv3746c | | PE/PPE protein |
| 173 | Rv3812 | | PE/PPE protein |
| 174 | Rv0638 | | The Sec-dependent general secretory pathway |
| 175 | Rv0732 | | The Sec-dependent general secretory pathway |
| 176 | Rv1008 | | The Sec-dependent general secretory pathway |
| 177 | Rv1224 | | The Sec-dependent general secretory pathway |
| 178 | Rv1821 | | The Sec-dependent general secretory pathway |
| 179 | Rv2093c | | The Sec-dependent general secretory pathway |
| 180 | Rv2094c | | The Sec-dependent general secretory pathway |
| 181 | Rv2586c | | The Sec-dependent general secretory pathway |
| 182 | Rv2587c | | The Sec-dependent general secretory pathway |
| 183 | Rv2588c | | The Sec-dependent general secretory pathway |
| 184 | Rv3240c | | The Sec-dependent general secretory pathway |

In the embodiment, the multiple genes used in MLST may preferably include at least 30S ribosome S1 to S21 (rpsAto rpsU), 50S ribosome L1 to L36 (rplAto rplF, rplI to rplU, rpmA to rpmJ), 5S ribosome RNA (rrf), 16S ribosome RNA (rrs), 23S ribosome RNA (rrl), rpoB, groEL2, gyrB, inhA, tlyA, katG, pncA, erm, eis, and embB, from the genes shown in Table 1. In addition to those genes, characteristic genes in NTM can be added according to NTM to be identified, in order to thereby improve accuracy of identification.

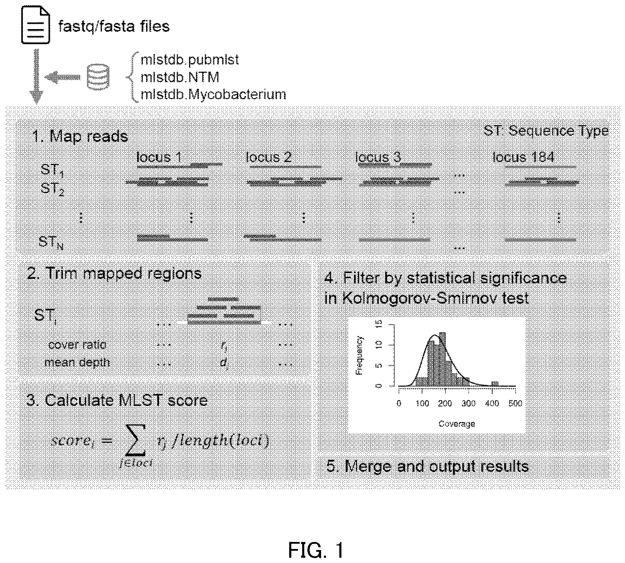

In the embodiment, the software for selecting, from the known NTM stored in the database, species or subspecies of NTM having most similar sequence information to the sequence information of NTM in the specimen, is not limited as long as the software can perform a workflow as shown in Figure 1. Particularly, mlstverse (https://github.com/ymatsumoto/mlstverse) developed by the inventors may be preferably used. As shown in Figure 1, the software firstly reads, from the database, sequence information in which assemblies of the 184 genes are mapped. As described above, the database stores sequence types for sequences of the 184 genes, corresponding to species or subspecies of NTM, and the number n of sequence types are shown in Figure 1. The software also reads sequence information acquired by the sequencing device and compares the sequence information with the sequences of each sequence type. The software performs alignment between the compared sequences to calculate a cover ratio and mean depth for the sequences. MLST score is subsequently calculated by using a mathematical expression shown in the item 3 of Figure 1. Statistical hypothesis test is then performed on the acquired data to see distribution of results obtained, for example, by Kolmogorov-Smirnov test as shown in Figure 1. The software then determines a sequence type having a highest MLST score from the sequence types ST1 to STN and outputs species or subspecies of NTM corresponding to the sequence type. In this instance, a list including multiple species or subspecies of NTM and MLST scores corresponding thereto may be output.

The software thus outputs species or subspecies of NTM in the specimen using the sequence information acquired from the sequencing device. In this respect, all of the sequence information in the 184 genes are used in the explanation above; however, the embodiment is not limited thereto and genes used can be appropriately selected from those genes.

### EXAMPLES

Examples are presented below for explaining in detail a system and a method for identifying species or subspecies of NTM in a specimen, according to the present disclosure. In Examples, conventional method using 16S ribosomal RNA gene sequences and the method according to the present disclosure are performed on clinical samples from 29 specimens to identify species and subspecies of NTM in the samples, and results obtained in the methods are compared. The methods and results are shown below. See also Emerging Microbes & Infections, Vol.8 (1), pp. 1043 to 1053 with reference to the methods in Examples.

### Samples

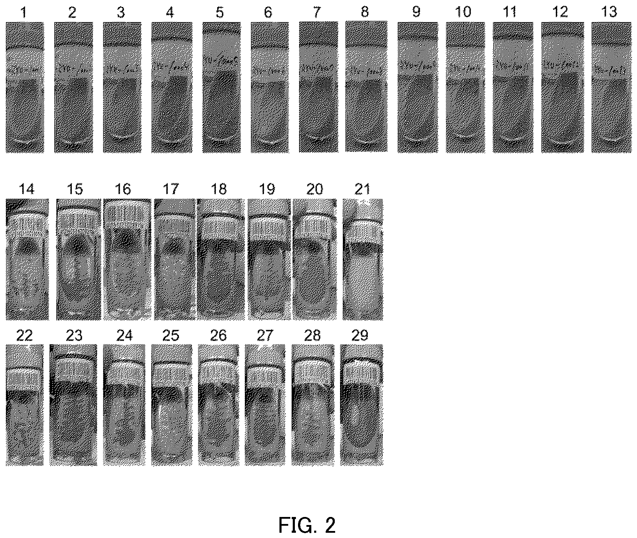

29 samples were isolated by a conventional method from clinical specimens obtained from patients in Ryukyu university hospital, and used as samples (Figure 2). The samples were seeded on 2% Ogawa medium and cultured at 35°C.

### Culturing of bacterial strains and extraction of DNA

The samples (bacterial strains) obtained as described above were cultured in a solid medium (19g of Middlebrook 7H10 agar (BD Difco), 5.0ml of glycerol, 895ml of distilled water, 100ml of Middlebrook OADC Enrichment (BD BBL), R agar (10.0g of Bacto peptone, BD Difco), 5.0g of yeast extract (BD Difco), 5.0g of malt extract (BD Difco), 5.0g of casamino acid (BD Difco), 2.0g of beef extract (BD Difco), 2.0g of glycerol, 50.0mg of Tween 80, 1.0g of MgSO4•7H2O), at 25°C, 30°C, 37°C, or 45°C in a time period of from 3 days to 7 weeks in an incubation oven (PIC-100 (As one).

The genomic DNAs of the bacterial strains were then extracted from colonies of the bacteria using DNeasy PowerSoil Kit (Qiagen).

### Constructing library and sequencing

Libraries were constructed with the extracted DNAs using Rapid Barcoding Kit provided by Oxford Nanopore Technologies. Experimental procedures were performed according to instructions included in the kit, and the libraries for MinION sequencer (Oxford Nanopore Technologies) were constructed by performing fragmentation of genomic DNA molecules, barcoding for identifying the samples, and adapter addition for sequencing. The library was loaded, and then subjected to a run, on MinION and sequence data in a fast 5 format serving as raw data were stored in a connected computer.

### Analysis in software

The output data in the fast 5 format were uploaded to a high performance computing server to perform a base calling process for converting to a fastq format serving as base sequence information. The sequence files subjected to the base calling process were processed by mlstverse to identify species of the bacteria. Names, MLST scores, and statistical values for each species of the bacteria were obtained and a species having a highest MLST score was determined as an identified result.

### Analysis using 16S ribosomal RNA sequence and results thereof

A conventional method for identifying species of bacteria using 16S ribosomal RNA sequences was performed as described below to compare with the results obtained from the method according to the present disclosure.

First, 16S ribosomal RNAs were amplified by polymerase chain reaction (PCR) using as a template the genomic DNAs extracted from the samples as described above. The following sequences are used as a primer.
27F: 5'-AGAGTTTGATCMTGGCTCAG-3'
1492R: 5' - TACGGYTACCTTGTTACGACTT-3'
3 3 7F: 5' -GACTCCTACGGGAGGCWGCAG-3'
1391R: 5'-GACGGGCGGTGTGTRCA-3'

PCR was performed using 2X KAPA HiFi HotStart ReadyMix (Roche) and 0.3 µM primer described above, at 95°C for 3 minutes, and then 30 cycles of 98°C for 20 seconds, 60°C for 15 seconds, and 72°C for 30 seconds. A final elongation was then performed at 72°C for 1 minute. The sequences of 16S ribosomal RNAs that were resulted products were determined and the sequences were compared with reference sequences of the Mycobacterium genus in a SILVA database by using blastn (https://blast.ncbi.nlm.nih.gov). As a result, species of the Mycobacterium genus showing a sequence identity equal to or higher than 98.7 % are presented in Table 2.

**Table 2**

| Sample No. | Species of bacteria | Sequence identity(%) |
|---|---|---|
| **1** | **Mycobacterium abscessus subsp. bolletii** | **99.13** |
| **1** | **Mycobacterium abscessus subsp. abscessus** | **99.13** |
| 1 | Mycobacterium chelonae | 98. 98 |
| **2** | **Mycobacterium fortuitum subsp. fortuitum** | **100.00** |
| **2** | **Mycobacterium fortuitum subsp. acetamidolyticum** | **100.00** |
| 2 | Mycobacterium senegalense | 99.53 |
| 2 | Mycobacterium houstonense | 99.53 |
| 2 | Mycobacterium farcinogenes | 99.53 |
| 2 | Mycobacterium conceptionense | 99.38 |
| 2 | Mycobacterium porcinum | 99.22 |
| 2 | Mycobacterium septicum | 99.14 |
| 2 | Mycobacterium neworleansense | 99.14 |
| 2 | Mycobacterium boenickei | 99.14 |
| 2 | Mycobacterium pallens | 98.99 |
| 2 | Mycobacterium peregrinum | 98.83 |
| 2 | Mycobacterium sphagni | 98.75 |
| 2 | Mycobacterium iranicum | 98.75 |
| 2 | Mycobacterium phocaicum | 98.75 |
| 2 | Mycobacterium mucogenicum | 98.75 |
| 2 | Mycobacterium setense | 99.13 |
| **3** | **Mycobacterium yongonense** | **100.00** |
| **3** | **Mycobacterium marseillense** | **100.00** |
| 3 | Mycobacterium vulneris | 99.92 |
| 3 | Mycobacterium colombiense | 99.85 |
| 3 | Mycobacterium chimaera | 99.85 |
| 3 | Mycobacterium bouchedurhonense | 99.77 |
| 3 | Mycobacterium intracellulare | 99.69 |
| 3 | Mycobacterium arosiense | 99.69 |
| 3 | Mycobacterium avium subsp. silvaticum | 99.54 |
| 3 | Mycobacterium avium subsp. avium | 99.38 |
| 3 | Mycobacterium avium subsp. paratuberculosis | 99.15 |
| 3 | Mycobacterium bohemicum | 99.23 |
| 3 | Mycobacterium seoulense | 99.15 |
| 3 | Mycobacterium paraseoulense | 99.15 |
| 3 | Mycobacterium riyadhense | 99.07 |
| 3 | Mycobacterium haemophilum | 99.07 |
| 3 | Mycobacterium nebraskense | 98.92 |
| 3 | Mycobacterium kansasii | 98.92 |
| 3 | Mycobacterium gastri | 98.92 |
| 3 | Mycobacterium lacus | 98.84 |
| 3 | Mycobacterium mantenii | 98.76 |
| **4** | **Mycobacterium abscessus subsp. bolletii** | **100.00** |
| **4** | **Mycobacterium abscessus subsp. abscessus** | **100.00** |
| 4 | Mycobacterium chelonae | 99.69 |
| 4 | Mycobacterium salmoniphilum | 99.30 |
| **5** | **Mycobacterium abscessus subsp. bolletii** | **100.00** |
| **5** | **Mycobacterium abscessus subsp. abscessus** | **100.00** |
| 5 | Mycobacterium chelonae | 99.69 |
| 5 | Mycobacterium salmoniphilum | 99.30 |
| **6** | **Mycobacterium abscessus subsp. bolletii** | **99.60** |
| **6** | **Mycobacterium abscessus subsp. abscessus** | **99.60** |
| 6 | Mycobacterium chelonae | 99.45 |
| 6 | Mycobacterium salmoniphilum | 99.05 |
| **7** | **Mycobacterium abscessus subsp. bolletii** | **99.60** |
| **7** | **Mycobacterium abscessus subsp. abscessus** | **99.60** |
| 7 | Mycobacterium chelonae | 99.44 |
| 7 | Mycobacterium salmoniphilum | 99.04 |
| **8** | **Mycobacterium abscessus subsp. bolletii** | **99.93** |
| **8** | **Mycobacterium abscessus subsp. abscessus** | **99.93** |
| 8 | Mycobacterium chelonae | 99.79 |
| 8 | Mycobacterium salmoniphilum | 99.44 |
| **9** | **Mycobacterium abscessus subsp. bolletii** | **99.44** |
| **9** | **Mycobacterium abscessus subsp. abscessus** | **99.44** |
| 9 | Mycobacterium chelonae | 99.28 |
| 9 | Mycobacterium salmoniphilum | 98.88 |
| **10** | **Mycobacterium abscessus subsp. bolletii** | **99.52** |
| **10** | **Mycobacterium abscessus subsp. abscessus** | **99.52** |
| 10 | Mycobacterium chelonae | 99.36 |
| 10 | Mycobacterium salmoniphilum | 98.96 |
| **11** | **Mycobacterium marseillense** | **100.00** |
| **11** | **Mycobacterium intracellulare** | **100.00** |
| **11** | **Mycobacterium chimaera** | **100.00** |
| **11** | **Mycobacterium yongonense** | **100.00** |
| **11** | **Mycobacterium vulneris** | **100.00** |
| 11 | Mycobacterium mantenii | 99.85 |
| 11 | Mycobacterium avium subsp. silvaticum | 99.85 |
| 11 | Mycobacterium seoulense | 99.71 |
| 11 | Mycobacterium scrofulaceum | 99.71 |
| 11 | Mycobacterium paraseoulense | 99.71 |
| 11 | Mycobacterium parascrofulaceum | 99.71 |
| 11 | Mycobacterium paraffinicum | 99.71 |
| 11 | Mycobacterium europaeum | 99.71 |
| 11 | Mycobacterium colombiense | 99.85 |
| 11 | Mycobacterium bohemicum | 99.71 |
| 11 | Mycobacterium arosiense | 99.85 |
| 11 | Mycobacterium lacus | 99.56 |
| 11 | Mycobacterium bouchedurhonense | 99.70 |
| 11 | Mycobacterium avium subsp. paratuberculosis | 99.41 |
| 11 | Mycobacterium avium subsp. avium | 99.41 |
| 11 | Mycobacterium palustre | 99.56 |
| 11 | Mycobacterium kansasii | 99.56 |
| 11 | Mycobacterium gastri | 99.56 |
| 11 | Mycobacterium riyadhense | 99.41 |
| 11 | Mycobacterium intermedium | 99.41 |
| 11 | Mycobacterium haemophilum | 99.41 |
| 11 | Mycobacterium malmoense | 99.26 |
| 11 | Mycobacterium szulgai | 99.12 |
| 11 | Mycobacterium nebraskense | 99.26 |
| 11 | Mycobacterium saskatchewanense | 98.82 |
| 11 | Mycobacterium kubicae | 99.51 |
| 11 | Mycobacterium bourgelatii | 99.48 |
| **12** | **Mycobacterium avium subsp. silvaticum** | **99.93** |
| 12 | Mycobacterium avium subsp. avium | 99.71 |
| 12 | Mycobacterium avium subsp. paratuberculosis | 99.56 |
| 12 | Mycobacterium colombiense | 99.64 |
| 12 | Mycobacterium vulneris | 99.56 |
| 12 | Mycobacterium bouchedurhonense | 99.56 |
| 12 | Mycobacterium marseillense | 99.34 |
| 12 | Mycobacterium yongonense | 99.34 |
| 12 | Mycobacterium arosiense | 99.34 |
| 12 | Mycobacterium intracellulare | 99.20 |
| 12 | Mycobacterium chimaera | 99.20 |
| 12 | Mycobacterium bohemicum | 99.05 |
| 12 | Mycobacterium riyadhense | 98.98 |
| 12 | Mycobacterium haemophilum | 98.91 |
| 12 | Mycobacterium seoulense | 98.91 |
| 12 | Mycobacterium paraseoulense | 98.91 |
| 12 | Mycobacterium kansasii | 98.76 |
| 12 | Mycobacterium gastri | 98.76 |
| **13** | **Mycobacterium avium subsp. silvaticum** | **100.00** |
| 13 | Mycobacterium avium subsp. avium | 99.85 |
| 13 | Mycobacterium avium subsp. paratuberculosis | 99.61 |
| 13 | Mycobacterium colombiense | 99.69 |
| 13 | Mycobacterium vulneris | 99.61 |
| 13 | Mycobacterium bouchedurhonense | 99.61 |
| 13 | Mycobacterium yongonense | 99.54 |
| 13 | Mycobacterium marseillense | 99.54 |
| 13 | Mycobacterium arosiense | 99.54 |
| 13 | Mycobacterium intracellulare | 99.38 |
| 13 | Mycobacterium chimaera | 99.38 |
| 13 | Mycobacterium bohemicum | 99.23 |
| 13 | Mycobacterium riyadhense | 99.15 |
| 13 | Mycobacterium haemophilum | 99.08 |
| 13 | Mycobacterium seoulense | 99.08 |
| 13 | Mycobacterium paraseoulense | 99.08 |
| 13 | Mycobacterium kansasii | 99.00 |
| 13 | Mycobacterium gastri | 99.00 |
| 13 | Mycobacterium nebraskense | 98.84 |
| 13 | Mycobacterium lacus | 98.84 |
| **14** | **Mycobacterium paragordonae** | **99.71** |
| 14 | Mycobacterium gordonae | 98.71 |
| **15** | **Mycobacterium paragordonae** | **99.21** |
| 15 | Mycobacterium asiaticum | 98.72 |
| **16** | **Mycobacterium lentiflavuα** | **99.64** |
| 16 | Mycobacterium triplex | 98.99 |
| 16 | Mycobacterium sherrisii | 98.92 |
| 16 | Mycobacterium simiae | 98.92 |
| 16 | Mycobacterium heidelbergense | 98.84 |
| 16 | Mycobacterium palustre | 98.70 |
| **17** | **Mycobacterium wolinskyi** | **99.50** |
| 17 | Mycobacterium mageritense | 98.93 |
| **18** | **Mycobacterium paragordonae** | **99.78** |
| 18 | Mycobacterium gordonae | 98.78 |
| **19** | **Mycobacterium lentiflavuα** | **99.71** |
| 19 | Mycobacterium triplex | 99.06 |
| 19 | Mycobacterium sherrisii | 98.99 |
| 19 | Mycobacterium simiae | 98.99 |
| 19 | Mycobacterium heidelbergense | 98.92 |
| 19 | Mycobacterium palustre | 98.78 |
| **20** | **Mycobacterium gastri** | **98.93** |
| 20 | Mycobacterium kansasii | 98.93 |
| 20 | Mycobacterium paraseoulense | 98.86 |
| 20 | Mycobacterium seoulense | 98.86 |
| **21** | **Mycobacterium wolinskyi** | **99.50** |
| 21 | Mycobacterium mageritense | 98.85 |
| **22** | **Mycobacterium paragordonae** | **98.77** |
| **23** | **Mycobacterium paragordonae** | **99.35** |
| 23 | Mycobacterium gordonae | 98.71 |
| **24** | **Mycobacterium paragordonae** | **99.13** |
| **25** | **Mycobacterium colombiense** | **99.71** |
| 25 | Mycobacterium bouchedurhonense | 99.64 |
| 25 | Mycobacterium vulneris | 99.50 |
| 25 | Mycobacterium yongonense | 99.49 |
| 25 | Mycobacterium arosiense | 99.43 |
| 25 | Mycobacterium marseillense | 99.35 |
| 25 | Mycobacterium avium subsp. Silvaticum | 99.28 |
| 25 | Mycobacterium chimaera | 99.21 |
| 25 | Mycobacterium intracellulare | 99.21 |
| 25 | Mycobacterium avium subsp. Avium | 99.07 |
| 25 | Mycobacterium avium subsp. Paratuberculosis | 98.92 |
| 25 | Mycobacterium bohemicum | 98.78 |
| 25 | Mycobacterium paraseoulense | 98.71 |
| 25 | Mycobacterium seoulense | 98.71 |
| **26** | **Mycobacterium europaeum** | **99.64** |
| 26 | Mycobacterium parascrofulaceum | 99.20 |
| 26 | Mycobacterium palustre | 98.92 |
| **27** | **Mycobacterium lentiflavum** | **99.00** |
| **28** | **Mycobacterium paragordonae** | **99.35** |
| **29** | **Mycobacterium lentiflavum** | **99.35** |
| 29 | Mycobacterium triplex | 98.70 |

As shown in Table 2, in most samples out of the 29 samples, multiple species of the bacteria showed significantly higher sequence identities equal to or higher 98.7 % in the conventional method. As a result, this revealed that it was difficult to identify species of the bacteria contained in the samples as single species in the conventional method.

### Results of analysis using the present disclosure

Next, Table 3 shows results obtained by constructing libraries, sequencing, and analyzing in software as explained above, using the genomic DNAs extracted from the 29 samples, according to the method of the present disclosure. Table 3 shows species of the bacteria having higher MLST scores in the samples and their MLST scores, which were obtained by analyzing in software.

**Table 3**

| Sample No. | Species of bacteria | Registration No. | MLST score |
|---|---|---|---|
| **1** | **Mycobacterium chelonae** | **ATCC 19237** | **0.94** |
| 1 | Mycobacterium abscessus | MAB_082312_2273 | 0.69 |
| **2** | **Mycobacterium fortuitum subsp. acetamidolyticum** | **JCM6368** | **0.97** |
| 2 | Mycobacterium fortuitum | E3337 | 0.78 |
| 2 | Mycobacterium fortuitum subsp. fortuitum | ATCC 6841 | 0.75 |
| 2 | Mycobacterium farcinogenes | DSM 43637 | 0.12 |
| **3** | **Mycobacterium yongonense** | **Asan 36527** | **0.87** |
| 3 | Mycobacterium chimaera | ZUERICH-2 | 0.51 |
| 3 | Mycobacterium intracellulare | MIN_052511_1280 | 0.46 |
| 3 | Mycobacterium paraintracellulare | MOTT64 | 0.31 |
| 3 | Mycobacterium avium subsp. avium | 2285 (S) | 0.29 |
| 3 | Mycobacterium indicus pranii | MTCC 9506 | 0.27 |
| **4** | **Mycobacterium abscessus subsp. abscessus** | **304** | **0.87** |
| 4 | Mycobacterium abscessus | PAP166 | 0.84 |
| 4 | Mycobacterium abscessus subsp. bolletii | MA 1948 | 0.64 |
| 4 | Mycobacterium fortuitum | Z58 | 0.58 |
| 4 | Mycobacterium abscessus subsp. massiliense | 75 | 0.42 |
| **5** | **Mycobacterium abscessus subsp.** | **214** | **0.97** |
| | **abscessus** | | |
| 5 | Mycobacterium abscessus | M94 | 0.96 |
| 5 | Mycobacterium fortuitum | Z58 | 0.72 |
| 5 | Mycobacterium abscessus subsp. bolletii | MA 1948 | 0.66 |
| 5 | Mycobacterium abscessus subsp. massiliense | 603 | 0.50 |
| **6** | **Mycobacterium chelonae** | **ATCC 19237** | **1.00** |
| 6 | Mycobacterium abscessus | MAB_082312_2273 | 0.74 |
| **7** | **Mycobacterium chelonae** | **ATCC 19237** | **1.00** |
| 7 | Mycobacterium abscessus | MAB_082312_2273 | 0.75 |
| **8** | **Mycobacterium abscessus subsp. bolletii** | **1S-151-0930** | **1.00** |
| 8 | Mycobacterium abscessus | FLAC004 | 1.00 |
| 8 | Mycobacterium abscessus subsp. massiliense | 177 | 1.00 |
| 8 | Mycobacterium abscessus subsp. abscessus | DJO-44274 | 0.99 |
| 8 | Mycobacterium fortuitum | Z58 | 0.26 |
| **9** | **Mycobacterium chelonae** | **D16R14** | **1.00** |
| 9 | Mycobacterium abscessus | MAB_082312_2273 | 0.69 |
| 9 | Mycobacterium stephanolepidis | NJB0901 | 0.11 |
| **10** | **Mycobacterium chelonae** | **35599** | **1.00** |
| 10 | Mycobacterium abscessus | MAB_082312_2273 | 0.69 |
| **11** | **Mycobacterium intracellulare** | **MIN_061107_1834** | **0.89** |
| 11 | Mycobacterium avium subsp. avium | 2285 (S) | 0.65 |
| 11 | Mycobacterium paraintracellulare | M0TT64 | 0.58 |
| 11 | Mycobacterium indicus pranii | MTCC 9506 | 0.55 |
| 11 | Mycobacterium chimaera | FLAC0067 | 0.46 |
| 11 | Mycobacterium yongonense | Asan 36912 | 0.32 |
| **12** | **Mycobacterium avium subsp. hominissuis** | **IH-945** | **0.81** |
| 12 | Mycobacterium avium | MAV_120709_2344 | 0.75 |
| 12 | Mycobacterium bouchedurhonense | DSM 45439 | 0.53 |
| 12 | Mycobacterium avium subsp. avium | ATCC 25291 | 0.50 |
| 12 | Mycobacterium timonense | CCUG 56329 | 0.50 |
| 12 | Mycobacterium avium subsp. silvaticum | ATCC 49884 | 0.45 |
| 12 | Mycobacterium avium subsp. paratuberculosis | 10-4404 | 0.39 |
| **13** | **Mycobacterium avium subsp. hominissuis** | **IH-945** | **0.82** |
| 13 | Mycobacterium avium | MAV_120709_2344 | 0.78 |
| 13 | Mycobacterium avium subsp. avium | 2285 (R) | 0.52 |
| 13 | Mycobacterium bouchedurhonense | DSM 45439 | 0.51 |
| 13 | Mycobacterium timonense | CCUG 56329 | 0.48 |
| 13 | Mycobacterium avium subsp. silvaticum | ATCC 49884 | 0.46 |
| 13 | Mycobacterium avium subsp. paratuberculosis | 10-4404 | 0.37 |
| **14** | **Mycobacterium gordonae** | **1275229.4** | **0.81** |
| 14 | Mycobacterium paragordonae | | 0.74 |
| **15** | **Mycobacterium gordonae** | **CTRI 14-8773** | **0.57** |
| 15 | Mycobacterium asiaticum | 1165133.8 | 0.14 |
| 15 | Mycobacterium paragordonae | | 0.14 |
| **16** | **Mycobacterium lentiflavum** | **CSUR P1491** | **1.00** |
| **17** | **Mycobacterium wolinskyi** | **ATCC 700010** | **0.92** |
| **18** | **Mycobacterium gordonae** | **1275229.4** | **0.94** |
| 18 | Mycobacterium paragordonae | | 0.25 |
| **19** | **Mycobacterium lentiflavum** | **CSUR P1491** | **1.00** |
| **20** | **Mycobacterium kansasii** | **14_15** | **0.55** |
| 20 | Mycobacterium gastri | DSM 43505 | 0.34 |
| 20 | Mycobacterium persicum | AFPC-000227 | 0.21 |
| **21** | **Mycobacterium wolinskyi** | **ATCC 700010** | **0.94** |
| **22** | **Mycobacterium gordonae** | **CTRI 14-8773** | **0.94** |
| **23** | **Mycobacterium gordonae** | **HMC_M15** | **0.97** |
| 23 | Mycobacterium paragordonae | | 0.25 |
| **24** | **Mycobacterium gordonae** | **CTRI 14-8773** | **0.94** |
| **25** | **Mycobacterium colombiense** | **CSURP297** | **0.83** |
| **26** | **Mycobacterium europaeum** | **CSUR P1344** | **0.97** |
| **27** | **Mycobacterium lentiflavum** | **CSUR P1491** | **1.00** |
| **28** | **Mycobacterium gordonae** | **1275229.4** | **0.61** |
| 28 | Mycobacterium paragordonae | | 0.14 |
| **29** | **Mycobacterium lentiflavum** | **CSUR P1491** | **1.00** |

As shown in Table 3, the mean value obtained from a highest MLST score in each of the 29 samples was a high value of 0.90. The mean value of the second highest scores was equal to or less than 0.50, which was greatly different than that of the highest scores. Thus, species of the bacteria having the highest score can be identified as the species of the bacteria in the sample. In addition, MLST scores differ between subspecies (subsp.) as seen, for example, in the samples No. 2 to 5, and thus subspecies of the bacterial strains can be also identified.

As described above, according to the present disclosure, species and subspecies of NTM in the specimen containing various NTM can be accurately identified.

## Claims

1. A system for identifying species or subspecies of non-tuberculous mycobacteria (NTM) in a specimen using MLST (multi-locus sequence typing), comprising:
a sequencing device for acquiring sequence information of multiple genes in the genomic sequence of NTM present in the specimen;
a database for storing sequence information of multiple genes in known NTM; and
a software for comparing the sequence information of the multiple genes acquired by the sequencing device with the sequence information of the multiple genes stored in the database to select, from the known NTM stored in the database, species or subspecies of NTM having most similar sequence information to the sequence information of NTM in the specimen,
wherein the multiple genes are selected from the group consisting of 184 genes shown in Table 1.

2. The system according to claim 1, wherein the multiple genes include 30S ribosome S1 to S21 (rpsAto rpsU), 50S ribosome L1 to L36 (rplAto rplF, rplI to rplU, rpmAto rpmJ), 5S ribosome RNA (rrf), 16S ribosome RNA (rrs), 23 S ribosome RNA (rrl), rpoB, groEL2, gyrB, inhA, tlyA, katG, pncA, erm, eis, and embB.

3. The system according to claim 1 or 2, wherein the database is mlstdb. NTM (https://github.com/ymatsumoto/mlstverse.Mycobacterium.db).

4. The system according to any of claims 1 to 3, wherein the software is mlstverse (http s: //github. com/ymatsumoto/ml stverse).

5. A method for identifying species or subspecies of non-tuberculous mycobacteria (NTM) in a specimen using MLST (multi-locus sequence typing), comprising the steps of:
performing sequencing of NTM present in the specimen to acquire sequence information of multiple genes in the genomic sequence of NTM;
comparing the acquired sequence information with sequence information of the multiple genes in known NTM stored in a predetermined database; and
selecting, from the known NTM stored in the database, species or subspecies of NTM having most similar sequence information to the sequence information of NTM in the specimen, by using a result of the comparison between the sequence information of the multiple genes,
wherein the multiple genes are selected from the group consisting of 184 genes shown in Table 1.

6. The method according to claim 5, wherein the multiple genes include 30S ribosome S1 to S21 (rpsAto rpsU), 50S ribosome L1 to L36 (rplAto rplF, rplI to rplU, rpmAto rpmJ), 5S ribosome RNA (rrf), 16S ribosome RNA (rrs), 23 S ribosome RNA (rrl), rpoB, groEL2, gyrB, inhA, tlyA, katG, pncA, erm, eis, and embB.

7. The method according to claim 5 or 6, wherein the database is mlstdb. NTM (https://github.com/ymatsumoto/mlstverse.Mycobacterium.db).

8. The method according to any of claims 5 to 7, wherein the software is mlstverse (http s: //github. com/ymatsumoto/ml stverse).
